# EUROPEAN PATENT APPLICATION

(11) **EP 4 721 772 A1**
(43) Date of publication of application: **08.04.2026**
(21) Application number: 24840111.9
(22) Date of filing: 11.07.2024
(51) Int. Cl.: A61L 27/54, A61L 27/18, A61L 27/36, A61L 27/52, A61L 27/34, A61L 27/56, A61L 27/58, D01D 5/00, D01D 1/09

(54) **BIO-IMPLANTABLE MICROFIBROUS IMPLANT AND METHOD FOR MANUFACTURING SAME**

(30) Priority: 13.07.2023 KR 20230090807
(71) Applicant: Cellknit Inc., Pohang-si, Gyeongsangbuk-do 37666 (KR)
(72) Inventor: LIM, Geun Bae, Gyeongju-si Gyeongsangbuk-do 38004 (KR); LEE, Jung Ho, Pohang-si Gyeongsangbuk-do 37673 (KR)
(74) Representative: Wichmann, Hendrik
(86) International application number: PCT/KR2024/009948
(87) International publication number: WO 2025/014301

(57) **Abstract**

The present disclosure relates to a bio-implantable microfiber graft and a method of manufacturing the same. A method of manufacturing a bio-implantable microfiber graft according to an exemplary embodiment of the present disclosure includes: manufacturing a porous nanofiber membrane based on a biocompatible polymer; and manufacturing one-dimensional porous microfibers by forming the porous nanofiber membrane in a twisted structure.

## Description

### CROSS-REFERENCE TO RELATED APPLICATION

This application claims the benefit of and priority to Korean Patent Application No. 10-2024-0091587, filed on July 11, 2024, and Korean Patent Application No. 10-2023-0090807, filed on July 13, 2023, the entire disclosure(s) of which is hereby incorporated herein by reference in its entirety.

### BACKGROUND

### Field

The present disclosure relates to a bio-implantable graft, and non-limitingly but more particularly, to a bio-implantable microfiber graft and a method of manufacturing the same which may effectively deliver drugs or cells into the body using a biocompatible microfiber.

### Description of Related Art

Development of materials for in vivo drug delivery and tissue regeneration plays a very important role in the fields of modern pharmaceuticals and life sciences, and such materials have been researched in the direction of delivering drugs to target tissues or organs to enhance drug efficacy, promote tissue regeneration, and reduce side effects. Biodegradable materials have a characteristic of slowly degrading over time, and when utilized for drug delivery and tissue regeneration, can improve the efficacy of drug therapy and reduce side effects. A drug delivery system using such materials has generally been developed in the form of scaffolds, which are solid framework materials, and hydrogels in a liquid form.

The scaffolds are implanted into lesion sites through surgical procedures and release drugs over an extended period while being gradually decomposed. However, they have limited surface area for drug incorporation, making effective drug delivery difficult due to insufficient capacity and drug release rates, and their inherent rigidity causes anatomical discomfort, and their mechanical stiffness does not match that of surrounding tissues or organs, consequently resulting in problems such as inflammation and damage to tissues or organs.

Additionally, biodegradable drug delivery systems in a filler form, such as hydrogels, involve injecting a liquid gel filler through a syringe, which gradually degrades and releases drugs over an extended period. However, due to structural instability and low mechanical strength of the liquid form, it is difficult to ensure consistent drug release, and the incorporated drugs are susceptible to degradation and instability.

### SUMMARY

As described above, among conventional materials for in vivo drug delivery and tissue regeneration, scaffolds have a limited surface area for drug incorporation, present difficulties in effective drug delivery, cause anatomical discomfort, and result in inflammation and damage to tissues or organs due to their inherent rigidity, and hydrogels, due to their low mechanical strength, make it difficult to ensure consistency of drug release and present problems of easy drug denaturation.

One object of the present disclosure for solving the aforementioned problems is to provide a bio-implantable microfiber graft which has a large surface area per unit volume for high drug diffusion efficiency and flexible mechanical properties for high compatibility with tissue, and maintains its shape even after time elapses following implantation into tissue to exhibit uniform drug release characteristics, and exhibits mechanically and chemically stable storage and transport performance during a process of loading and releasing drugs.

Another object of the present disclosure for solving the aforementioned problems is to provide a method of manufacturing a bio-implantable microfiber graft that simultaneously achieves a high drug loading capacity and a sustained drug release mechanism through a microporous structure that facilitates drug absorption and release, and enables drug delivery and tissue regeneration promotion in response to various environmental conditions and stimuli.

However, it is to be understood that the technical problem to be solved by the present disclosure is not limited to the above problems and may be variously extended in a range which does not depart from the spirit and scope of the present disclosure.

In order to achieve the above-described object, in an aspect, provided is a method of manufacturing a bio-implantable microfiber graft which may include: manufacturing a porous nanofiber membrane based on a biocompatible polymer; and manufacturing one-dimensional porous microfibers by forming the porous nanofiber membrane in a twisted structure.

According to an aspect, the porous nanofiber membrane or the microfibers may be coated with perylene.

The method of manufacturing the bio-implantable microfiber graft according to an exemplary embodiment of the present disclosure may further include forming nanoelectrodes by depositing a conductive material on the porous nanofiber membrane.

According to an aspect, the nanoelectrode may be formed in a two-dimensional multi-array structure on a surface of the porous nanofiber membrane, and a plurality of nanoelectrodes included in the multi-array structure may be arranged spaced apart from each other in a longitudinal direction of the microfibers when the porous nanofiber membrane is manufactured into microfibers.

The method of manufacturing the bio-implantable microfiber graft according to an exemplary embodiment of the present disclosure may further include forming the plurality of nanofiber membranes into a nanofiber bundle.

According to an aspect, the nanofiber bundle may have a core having a first stiffness and a shell surrounding the core, which has a second stiffness different from the first stiffness.

According to an aspect, the core of the nanofiber bundle may be formed of Polylactic Acid (PLA), and the shell may be formed of Polycaprolactone (PCL).

According to an aspect, the nanofiber bundle may be formed of hook-shaped nanofibers.

According to an aspect, the nanofiber bundle may include a main fiber and microfibers connected thereto.

According to an aspect, the nanofiber bundle may be formed by mixing multi-layered nanofiber membranes and forming them in a rolled or twisted form.

According to an aspect, the nanofiber membrane may be manufactured as a nanofiber membrane having an area equal to or greater than a predetermined critical area through multi-nozzles, so that the microfiber may have a length equal to or greater than a predetermined critical length.

The method of manufacturing a bio-implantable microfiber graft according to an exemplary embodiment of the present disclosure may further include loading a stimuli-responsive hydrogel onto the nanoelectrode.

According to an aspect, the stimuli-responsive hydrogel may contain a drug.

According to an aspect, the stimuli-responsive hydrogel may be responsive to pH to release the drug.

According to an aspect, the stimuli-responsive hydrogel may be responsive to an ion concentration to release the drug.

According to an aspect, the stimuli-responsive hydrogel may be responsive to a glucose concentration to release the drug.

According to an aspect, the stimuli-responsive hydrogel may be responsive to an antigen concentration to release the drug.

The method of manufacturing the bio-implantable microfiber graft according to an exemplary embodiment of the present disclosure may further include loading functional stem cells onto the porous nanofiber membrane.

According to an aspect, a cross-section of the microfiber formed by the porous nanofiber membrane loaded with the functional stem cells may be formed such that density and strength gradually change from one side to the other side.

According to an aspect, a decellularized extracellular matrix-based hydrogel of a target organ may be loaded onto at least a portion of the surface of the microfiber.

In another aspect, provided is a bio-implantable microfiber graft which may include one-dimensional microfibers manufactured by forming a porous nanofiber membrane, which is formed based on a biocompatible polymer, into a nanofiber bundle and then forming the nanofiber bundle into a twisted structure.

The disclosed technology may have the following effects. However, since it is not meant that a specific exemplary embodiment should include all of the following effects or merely include the following effects, the scope of the disclosed technology is not to be construed as being limited thereby.

According to the bio-implantable microfiber graft and a method of manufacturing the same according to an exemplary embodiment of the present disclosure described above, by molding a nanofiber membrane in a twisted structure to manufacture microfibers, it is possible to provide a bio-implantable microfiber graft having excellent mechanical strength while being flexible, and having excellent drug release characteristics due to a large surface area per unit volume.

In addition, by forming electrodes from porous nanofiber membranes having a large surface area per unit volume, the electrode exhibits excellent sensing rate, and by incorporating stimuli-responsive hydrogels that can respond to various stimuli, various drugs can be effectively released without structural changes to the microfiber graft.

Furthermore, the bio-implantable microfiber graft mimics body tissue and incorporates stem cells into a porous nanofiber membrane to effectively cultivate cells and optimize tissue regeneration, and by incorporating a decellularized extracellular matrix-based hydrogel, can effectively regenerate target tissues or organs without causing immune response problems.

### BRIEF DESCRIPTION OF THE DRAWING

FIG. 1 is a schematic diagram illustrating a process of manufacturing a bio-implantable microfiber graft according to an exemplary embodiment of the present disclosure.
FIG. 2 is a diagram exemplarily illustrating the thickness and the twist structure of microfibers.
FIG. 3 is a schematic diagram illustrating a process of manufacturing a large-area nanofiber membrane according to an exemplary embodiment of the present disclosure.
FIG. 4 is a photograph illustrating coating of perylene on microfibers.
FIG. 5 exemplarily illustrates an implantation process of a bio-implantable microfiber graft according to an exemplary embodiment of the present disclosure.
FIG. 6 is a photograph illustrating an implantation result of the bio-implantable microfiber graft.
FIG. 7 exemplarily illustrates a nanoelectrode-forming process of a nanofiber membrane according to an exemplary embodiment of the present disclosure.
FIG. 8 is a schematic diagram of a two-dimensional multi-array electrode formed on the nanofiber membrane.
FIG. 9 exemplarily illustrates loading of a responsive hydrogel onto a nanoelectrode according to an exemplary embodiment of the present disclosure.
FIG. 10 is a schematic diagram for schematically describing a response of the responsive hydrogel of FIG. 9.
FIG. 11 exemplarily illustrates a nanofiber membrane according to a density variation.
FIG. 12 is a schematic diagram exemplarily illustrating voltage characteristics according to density changes of a nanofiber membrane having nanoelectrodes formed thereon.
FIG. 13 exemplarily illustrates stem cell culture and material diffusion on the nanofiber membrane according to an exemplary embodiment of the present disclosure.
FIG. 14 is a schematic diagram exemplarily illustrating a multi-layered structure of the bio-implantable microfiber graft.
FIG. 15 exemplarily illustrates an operation of the bio-implantable microfiber graft according to an exemplary embodiment of the present disclosure.

### DETAILED DESCRIPTION

The present disclosure may have various modifications and various exemplary embodiments and specific exemplary embodiments will be illustrated in the drawings and described in detail.

However, this does not limit the present disclosure to specific exemplary embodiments, and it should be understood that the present disclosure covers all the modifications, equivalents and substitutes included within the spirit and technical scope of the present disclosure.

Terms such as "first," "second," etc. may be used to describe various components, but the components should not be limited by these terms. These terms are used only for the purpose of distinguishing one element from another. For example, a first component may be referred to as a second component, and similarly, the second component may be referred to as the first component without departing from the scope of the present disclosure. A term 'and/or' includes a combination of a plurality of associated disclosed items or any item of the plurality of associated disclosed items.

It should be understood that, when it is described that a constituent element is "connected to" or "accesses" another constituent element, the constituent element may be directly connected to or access the other constituent element or a third constituent element may be present therebetween. In contrast, when it is described that a constituent element is "directly connected to" or "directly accesses" another constituent element, it is understood that no constituent element is present between the constituent element and another constituent element.

Terms used in the present application are used only to describe specific exemplary embodiments, and are not intended to limit the present disclosure. Singular expressions include plural expressions unless the context clearly indicates otherwise. In this application, terms such as "comprise" or "have" are intended to designate that features, numbers, steps, operations, components, parts, or combinations thereof described in the specification exist, and should be understood as not precluding in advance the existence or addition possibility of one or more other features, numbers, steps, operations, components, parts, or combinations thereof.

Unless otherwise defined, all terms used herein, including technical or scientific terms, have the same meaning as commonly understood by those skilled in the art to which the present disclosure pertains. Terms which are defined in a generally used dictionary should be interpreted to have the same meaning as the meaning in the context of the related art, and are not interpreted as an ideal meaning or excessively formal meanings unless clearly defined in the present application.

Hereinafter, preferred exemplary embodiments of the present disclosure will be described in more detail with reference to the accompanying drawings. In describing the present disclosure, the same reference numerals are used for the same components in the drawings and a duplicated description of the same components will be omitted for facilitating overall understanding of the present disclosure.

As discussed above, the conventional drug delivery and tissue regeneration system using solid or liquid-state materials for tissue regeneration and in vivo drug delivery had a difficulty in effective drug delivery and causes anatomical discomfort, and the mechanical stiffness does not match that of surrounding tissues or organs, which could consequently cause problems such as inflammation and damage to tissues or organs, and had problems of instability in which it was difficult to ensure consistency of drug release and the accompanying drugs were easily denatured.

The bio-implantable microfiber graft according to an exemplary embodiment of the present disclosure is intended to solve the aforementioned problems, and by manufacturing a porous nanofiber membrane as one-dimensional microfibers, it is possible to provide a bio-implantable microfiber graft having excellent mechanical strength while being flexible and having excellent drug release characteristics due to a large surface area per unit volume, and accordingly, the bio-implantable microfiber graft may be used as a surgical suture. In addition, due to electrode formation, a stimuli-responsive hydrogel, a decellularized extracellular matrix-based hydrogel, and the loading of stem cells on the bio-implantable microfiber graft, drugs may be effectively released and effective cell culture and tissue regeneration may be achieved without immune response problems to tissues or organs.

Hereinafter, a bio-implantable microfiber graft according to an exemplary embodiment of the present disclosure and a method of manufacturing the same will be described in more detail with reference to the drawings.

Hereinafter, in the present disclosure, the "bio-implantable microfiber graft" may refer to any fiber structure having a diameter generally ranging from nanometer to micrometer scale that may be implanted into body tissues to perform various functions, but it should be understood that the unit of the microfiber is not limited thereto.

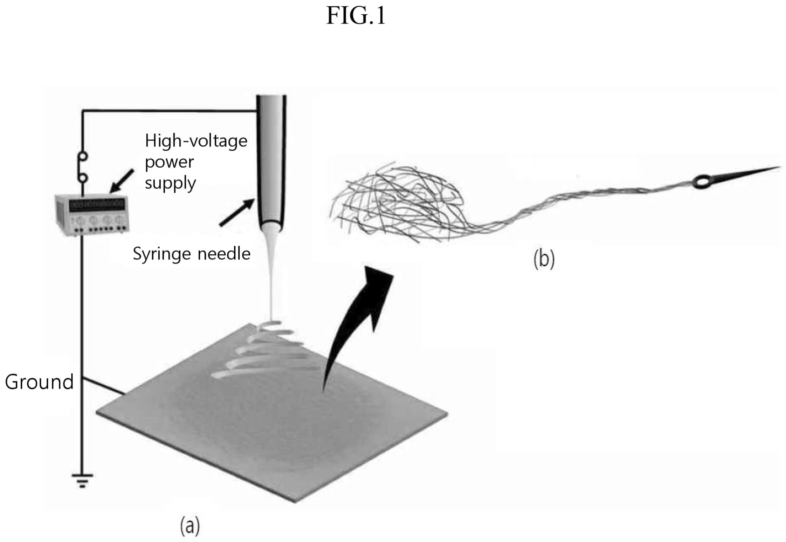

FIG. 1 is a schematic diagram illustrating a process of manufacturing a bio-implantable microfiber graft according to an exemplary embodiment of the present disclosure. The method of manufacturing a bio-implantable microfiber graft according to an exemplary embodiment of the present disclosure may first manufacture a porous nanofiber membrane based on a biocompatible polymer, as illustrated in (a) of FIG. 1. As a non-limiting example, it should be noted that the porous nanofiber membrane may be manufactured by electrospinning the biocompatible polymer, but is not limited thereto. To this end, the biocompatible polymer is dissolved in a solvent to prepare a biocompatible polymer solution having a desired viscosity and concentration, which is then supplied to a syringe needle-type nozzle. After positioning a syringe needle toward a collector plate, when a high voltage is applied between the syringe needle and the collector plate by a high voltage source, an electric field is generated, and a polymer solution is drawn out in the form of fine fibers by the resulting electric force. The fibers are formed into nanofibers as an internal solvent evaporates, and the nanofibers emitted from the syringe needle are accumulated on the collector plate, thereby manufacturing the porous nanofiber membrane. Such a porous nanofiber membrane may achieve an optimal final product structure by controlling the concentration and viscosity of the polymer solution and the electrospinning environment conditions (voltage, temperature, and humidity).

Subsequently, as illustrated in (b) of FIG. 1, the porous nanofiber membrane may be formed into a twisted structure to manufacture one-dimensional porous microfibers. A nanofiber membrane composed of very fine nanofibers may be structurally very weak in mechanical strength. However, a two-dimensional nanofiber membrane composed of one-dimensional nanofibers has a porous structure from a microscopic perspective and is well known as a material that mimics an extracellular matrix of biological tissues. Therefore, in order to increase the mechanical strength of such a porous nanofiber membrane and increase the surface area of the nanofiber membrane according to the porous structure for implantation into biological tissues, the porous nanofiber membrane may be formed in a twisted structure as illustrated in (b) of FIG. 1, and converted into one-dimensional porous microfibers.

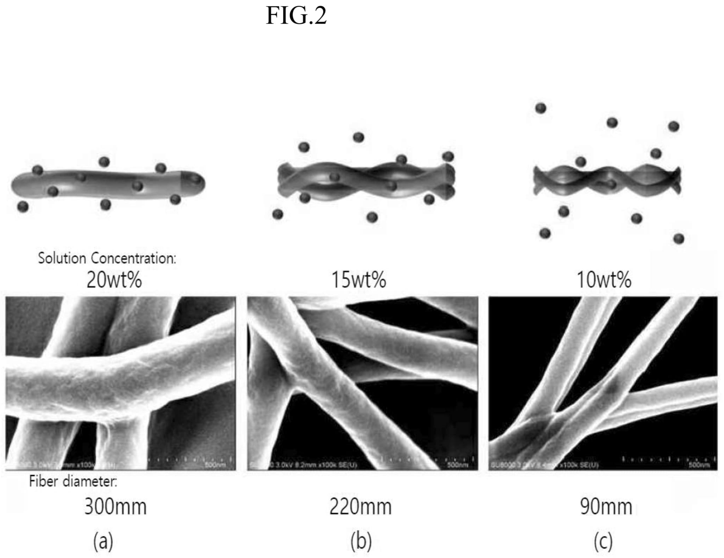

FIG. 2 is a diagram exemplarily illustrating the thickness and the twist structure of microfibers. The twist structure for enabling the nanofiber membrane to better withstand greater tensile forces may vary according to various design approaches that may optimize the mechanical strength and flexibility of the microfibers. In general, as the degree of twist of the fiber increases, the strength of the fiber increases but the flexibility decreases, so an experiment was conducted for optimal design of the twist structure according to thickness control of electrospun nanofibers to derive a maximum twist structure limit of the nanofiber membrane that may maintain the flexibility of the microfibers.

(a) of FIG. 2 illustrates a nanofiber formed with a diameter of 300 nm by controlling the concentration of a polymer solution for manufacturing a nanofiber membrane to 20 wt%; (b) of FIG. 2 illustrates a nanofiber formed with a diameter of 220 nm by controlling the concentration of the polymer solution to 15 wt%, and (c) of FIG. 2 illustrates a nanofiber formed with a diameter of 90 nm by controlling the concentration of the polymer solution to 10 wt%. When the nanofibers are twisted, stress is distributed among the twisted fibers when a load is applied, which allows each fiber to withstand a certain load, and the load is evenly distributed, thereby improving the tensile strength of the entire twisted fiber. Further, the nanofibers support each other while transmitting forces in opposite directions in the twisted form, and friction occurs as the twisted fibers cross and contact each other, which may contribute to withstanding a tensile force.

As described above, fibers having increased tensile strength and durability through a twisted structure of fibers may be formed with reduced thickness of individual fibers due to the increased tensile strength and durability, and a ratio of surface area per unit volume may be maximized through bundles of twisted structures of even finer fibers. Such nanofibers have an extremely increased surface area per unit volume, so that a passive diffusion behavior of particles within the fibers may be greatly improved to increase reaction efficiency. The passive diffusion behavior of the particles is schematically illustrated in FIG. 2. In an aspect, microfibers manufactured from such nanofiber membranes may function as a drug delivery platform by converting the release of particles such as drugs into time-series data, and may also be utilized to include fluorescent particles in drugs to monitor precise particle diffusion behavior.

Although not illustrated, a method of manufacturing a bio-implantable microfiber graft according to an exemplary embodiment of the present disclosure may form a plurality of nanofiber membranes into a nanofiber bundle. In an aspect, the nanofiber bundle may be controlled to form a shape of the nanofiber bundle by changing a condition of the polymer solution and electrospinning, etc., and a diameter, arrangement, bundling, etc., of the nanofibers may be controlled, and, for example, the nanofiber membranes formed in a multi-layered form may be formed by mixing and rolling or twisting.

According to an aspect, the nanofiber bundle may have a core having a first stiffness and a shell surrounding the core, which has a second stiffness different from the first stiffness. As a non-limiting example, the second stiffness may be lower than the first stiffness, but is not limited thereto. In an aspect, for example, the core of the nanofiber bundle may be formed of Polylactic Acid (PLA), and the shell may be formed of Polycaprolactone (PCL). The PLA and the PCL are representative biodegradable nanofiber materials, wherein the PLA in the core has relatively superior biodegradability and faster biodegradation rate compared to the PCL in the shell, and has higher stiffness and lower elongation. Further, the PCL in the shell has relatively lower biodegradability and a slower biodegradation rate compared to the PLA in the core, and has higher flexibility and excellent elongation while having lower stiffness characteristics. That is, the core of the nanofiber bundle is made of a rigid material to maintain strength while the shell is made of a flexible material to facilitate formation of the twisted structure, and such a structure may be effective in optimizing the flexibility and mechanical strength of the microfiber. In an aspect, the nanofiber bundle may be composed of different materials other than the PLA and the PCL for the core and shell, and the different materials may be any materials as long as they are bio-implantable materials, so there is no limitation on the type.

In an aspect, the nanofiber bundle may also be formed of hook-shaped nanofibers. Such hook-shaped nanofibers have fiber ends formed in a hook shape to increase the surface area and may provide a mechanical interlocking effect, thereby exhibiting excellent effects in improving cell attachment and proliferation due to enhanced adhesion and friction during bio-implantation. According to an aspect, the nanofiber bundle may also include main fibers and microfibers connected thereto. In such a composite structure nanofiber bundle, thick fibers forming a main structure have high strength and serve as structural supports, while fine fibers connected to the main fibers may increase the surface area to improve functionalities such as drug release or flexibility. In an aspect, the nanofiber bundle may also be manufactured in a form where multi-layered nanofiber membranes are mixed and rolled or twisted without distinction between the core and the shell.

According to an aspect, the bio-implantable microfiber graft according to an exemplary embodiment of the present disclosure may include a one-dimensional microfiber manufactured by forming a porous nanofiber membrane formed by electrospinning a biocompatible polymer as described above into nanofiber bundles and then forming the nanofiber bundles into the twisted structure. Such one-dimensional microfibers are formed by forming the porous nanofiber membrane into the nanofiber bundles and then forming the nanofiber bundles into the twisted structure, thereby having high strength and specific surface area, so that the one-dimensional microfibers may have sufficient strength and functionality to be implanted into the body, enabling the microfibers themselves to be used as surgical sutures.

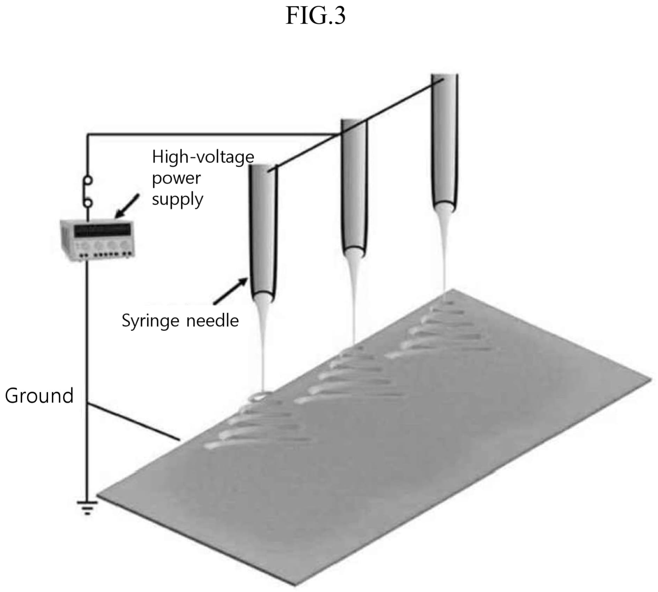

FIG. 3 is a schematic diagram illustrating a process of manufacturing a large-area nanofiber membrane according to an exemplary embodiment of the present disclosure. According to an aspect, the nanofiber membrane is manufactured as a nanofiber membrane having an area equal to or greater than a predetermined critical area through multi-nozzles, so that the microfiber may have a length equal to or greater than a predetermined critical length. As described above with respect to FIG. 2, in order to use the microfiber according to the present disclosure as surgical sutures, microfibers having a length equal to or greater than a critical length that may be used as surgical sutures are required. To this end, as shown in FIG. 3, a large-area nanofiber membrane having an area equal to or greater than a critical area and ensuring uniformity may be manufactured by controlling the electric field of multi-nozzles through an electrospinning technique configured based on syringe needle-type multi-nozzles. The large-area nanofiber membrane may be converted into a microfiber membrane having, for example, a length of 30 cm or more by forming nanofiber bundles and twisted structures as described above, thereby making it possible to use the microfiber membrane as surgical sutures.

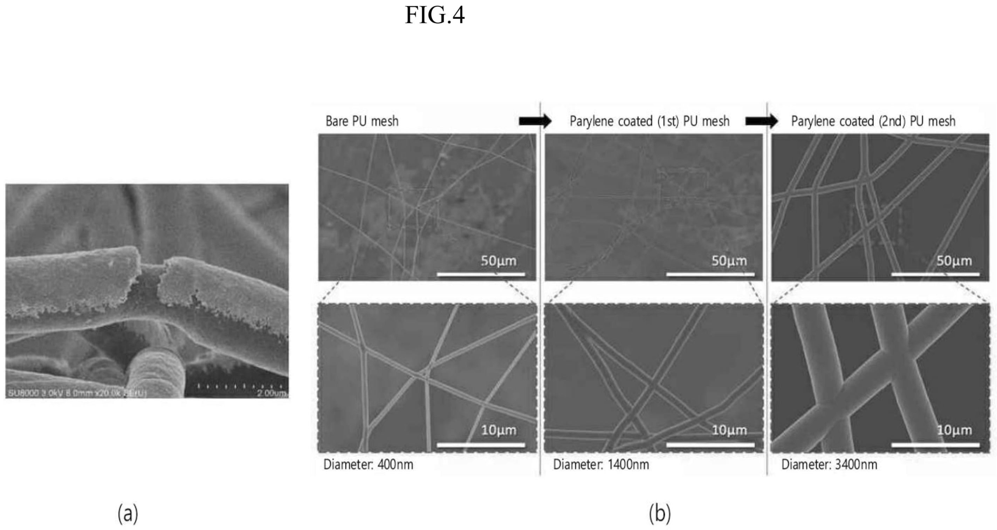

FIG. 4 is a photograph illustrating coating of perylene on microfibers. According to an aspect of the present disclosure, the porous nanofiber membrane or microfibers may be coated with perylene. The parylene is a material known as a bioinert material that is chemically and biologically very stable, exhibiting excellent biocompatibility and low tissue responsiveness, while demonstrating electrically excellent insulating properties and being mechanically flexible yet having high strength. Nanofiber membranes not coated with such parylene may have unjoined portions between nanofibers as shown in (a) of FIG. 4. When unjoined spaces occur between nanofibers, a distance between nanofibers may increase during subsequent metal deposition for electrode formation, potentially causing electrode breakage. Therefore, to integrate the junctions of nanofibers, the parylene may be coated as shown in (b) of FIG. 4. (b) of FIG. 4 shows an initial PU nanofiber membrane, a PU nanofiber membrane with a primary parylene coating applied, and a PU nanofiber membrane with a secondary parylene coating applied. Comparing (a) and (b) of FIG. 4, an integrated appearance of the nanofiber junctions may be confirmed. Although FIG. 4 describes parylene coating for PU nanofiber membranes, the parylene coating is not limited to materials and thickness, and it should be understood that parylene coating may be applied to nanofiber membranes, nanofiber bundles, or microfiber state, respectively, to integrate the junctions between nanofibers.

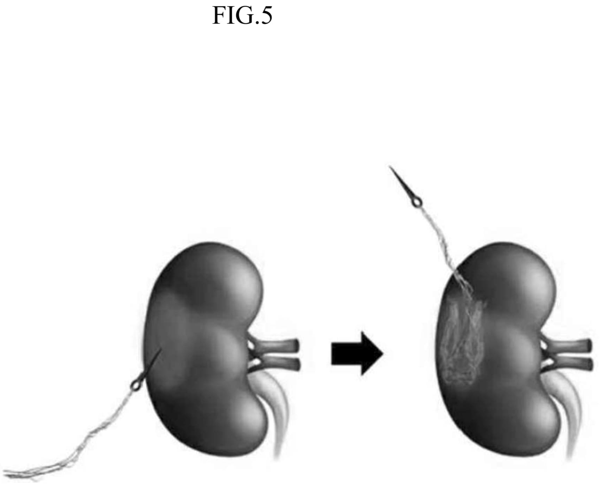

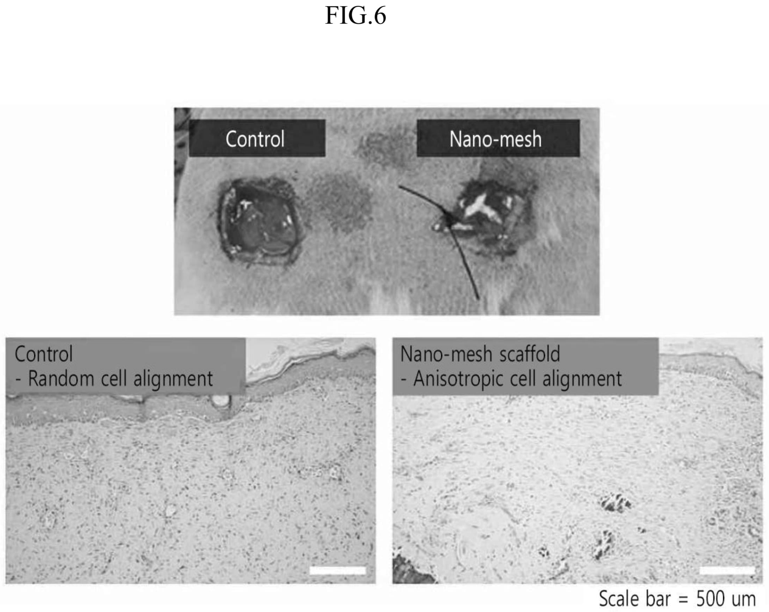

FIG. 5 exemplarily illustrates an implantation process of a bio-implantable microfiber graft according to an exemplary embodiment of the present disclosure, and FIG. 6 is a photograph illustrating an implantation result of the bio-implantable microfiber graft. The bio-implantable microfiber graft according to the present disclosure may be implanted into biological tissue by being combined with a surgical needle in a structure similar to surgical sutures as shown in FIG. 5. For example, as shown in FIG. 5, microfibers may be flexibly implanted into biological tissue like surgical sutures through three or more back-and-forthmovements at an anticipated lesion site of an organ, and since this flexibility has high compatibility with organ flexibility, more effective cell engraftment effects may be obtained during subsequent cell engraftment. A result of such cell engraftment is shown in FIG. 6. Referring to FIG. 6, a result of implanting microfibers formed from nanofiber membranes as surgical sutures may be confirmed. Compared to a control group on the left, the microfiber graft on the right shows a reduced size of the lesion site, and since cellular components recover their original directionality and show anisotropic cell alignment, it may be confirmed that tissue regeneration is being effectively achieved.

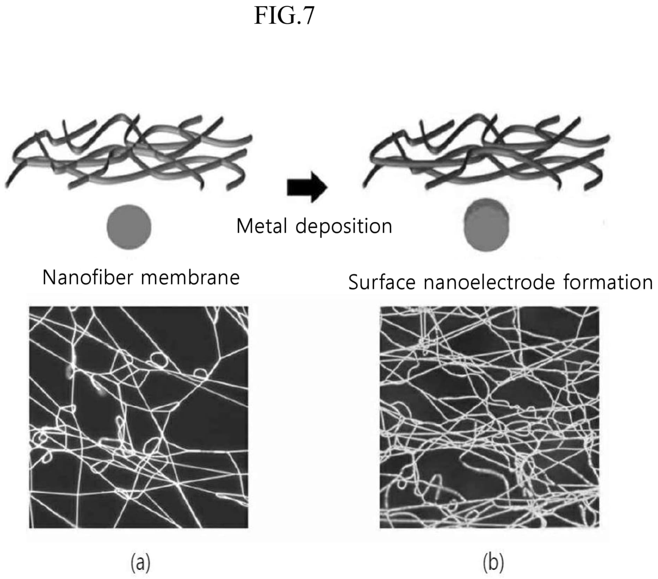

FIG. 7 exemplarily illustrates a nanoelectrode formation process of a nanofiber membrane according to an exemplary embodiment of the present disclosure. The method of manufacturing the bio-implantable microfiber graft according to an exemplary embodiment of the present disclosure formed as described above may further include forming nanoelectrodes by depositing a conductive material on the porous nanofiber membrane. As a non-limiting example, the conductive material may include metals or conductive polymers. For example, conductive metals may include, but are not limited to, gold, silver, copper, platinum deposition, or silver nanowires, gold nanoparticles. Additionally, conductive polymers may include, for example, PEDOT:PSS, but are not limited thereto. As described above with respect to FIG. 4, after loading parylene, which is a bioinert material, on the surface of the nanofiber membrane, nanoelectrodes may be formed by depositing the conductive materials on the surface of such nanofiber membrane as illustrated in FIG. 7. Such conductive materials may be any metals having excellent biocompatibility and electrochemical stability, and may be selected from the group consisting of, for example, gold (Au), platinum (Pt), titanium (Ti), iridium (Ir), silver (Ag), aluminum (Al), iron (Fe), carbon, and the like. Such conductive metals may be deposited on the nanofiber membrane by known techniques capable of depositing metals on polymer fibers, such as inorganic vacuum deposition, sputter deposition, evaporation deposition, electron beam physical vapor deposition, pulsed laser deposition, chemical vapor deposition, wet coating, and the like.

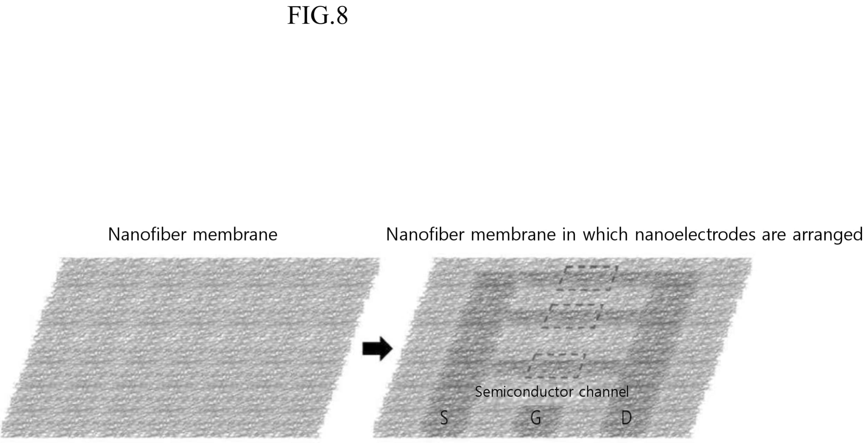

FIG. 8 is a schematic diagram of a two-dimensional multi-array electrode formed on the nanofiber membrane. As illustrated in FIG. 8, nanoelectrodes according to an aspect may be formed in a two-dimensional multi-array structure on the surface of the porous nanofiber membrane. A plurality of nanoelectrodes included in the multi-array structure may be arranged spaced apart from each other along a longitudinal direction of the microfibers when the porous nanofiber membrane is manufactured into microfibers. More specifically, when the nanoelectrodes have the multi-array structure and become one-dimensional microfibers, the plurality of nanoelectrodes may be arranged along a longitudinal fiber direction, so quantification of changes at specific positions (for example, physical deformation, defects on the nanofiber membrane surface, and changes in charge intensity in the surrounding environment) may be possible through electrical signal detection at specific positions. Further, spatial resolution may be enhanced through the multi-array structure of the nanoelectrodes to selectively monitor signals from specific regions, and multiple nanoelectrodes may be integrated to secure high channel density, thereby making it possible to simultaneously sense more information.

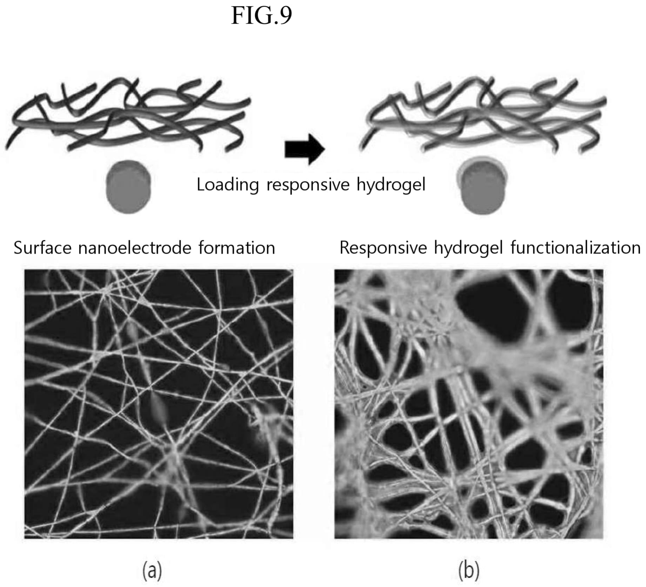

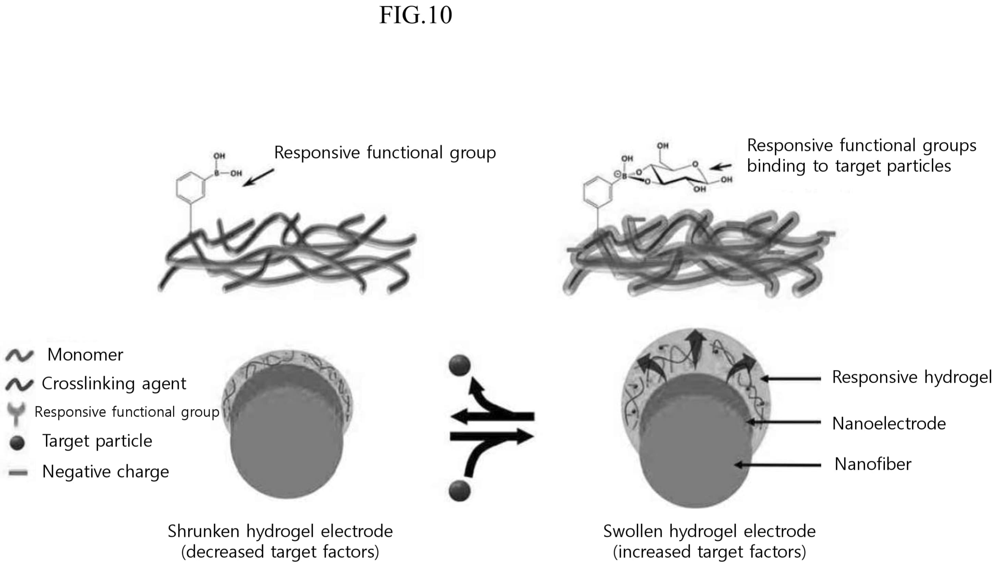

FIG. 9 exemplarily illustrates loading of a responsive hydrogel onto a nanoelectrode according to an exemplary embodiment of the present disclosure and FIG. 10 is a schematic diagram for schematically describing a response of the responsive hydrogel of FIG. 9. The method of manufacturing a bio-implantable microfiber graft according to an exemplary embodiment of the present disclosure may further include loading a stimuli-responsive hydrogel onto the nanoelectrode. A drug may be contained within the stimuli-responsive hydrogel.

More specifically, the hydrogel is a hydrophilic polymer material with a high moisture content, and in order to allow the stimuli-responsive polymer to enter the hydrogel, a stimuli-responsive polymer monomer is crosslinked with the hydrogel through light or heat to form a three-dimensional structure. The stimuli-responsive hydrogel may then be loaded on the nanoelectrode formed on the surface of the nanofiber membrane, as illustrated in FIG. 9. In an aspect, such stimuli-responsive hydrogel may be integrated with the nanoelectrode, for example, by using dip coating or an electrical method, and thus expansion and contraction of the stimuli-responsive hydrogel may be quantified in real time through the nanoelectrode.

More specifically, referring to FIG. 10, in an aspect, the stimuli-responsive hydrogel is synthesized by introducing a monomer that induces the expansion or contraction of the hydrogel by varying a surface charge according to external environmental changes, and may also include a crosslinking agent, reactive functional groups, and a drug inside. When a bio-implantable microfiber graft loaded with an externally stimuli-responsive hydrogel according to the present disclosure is implanted into the body, when the concentration of, for example, pH, glucose, ions, and antigens increases in a body environment, reactive functional groups bind to their target particles and the hydrogel expands, and in an opposite case, the hydrogel contracts. When the hydrogel expands, the drug inside the stimuli-responsive hydrogel is released, which may cause a reaction to a stimulus.

In an aspect, when the reactive functional groups bind to pH target particles, the stimuli-responsive hydrogel may react to a pH concentration and release the drug when pH increases. In an aspect, when the reactive functional groups bind to ion target particles, the stimuli-responsive hydrogel may react to an ion concentration and release the drug when a target ion concentration increases. In an aspect, when the reactive functional groups bind to glucose target particles, the stimuli-responsive hydrogel may react to a glucose concentration and release the drug (for example, insulin) when the glucose concentration increases. In an aspect, when the reactive functional groups bind to antigen target particles, the stimuli-responsive hydrogel may react to an antigen concentration and release the drug (for example, antibodies) when the antigen concentration increases.

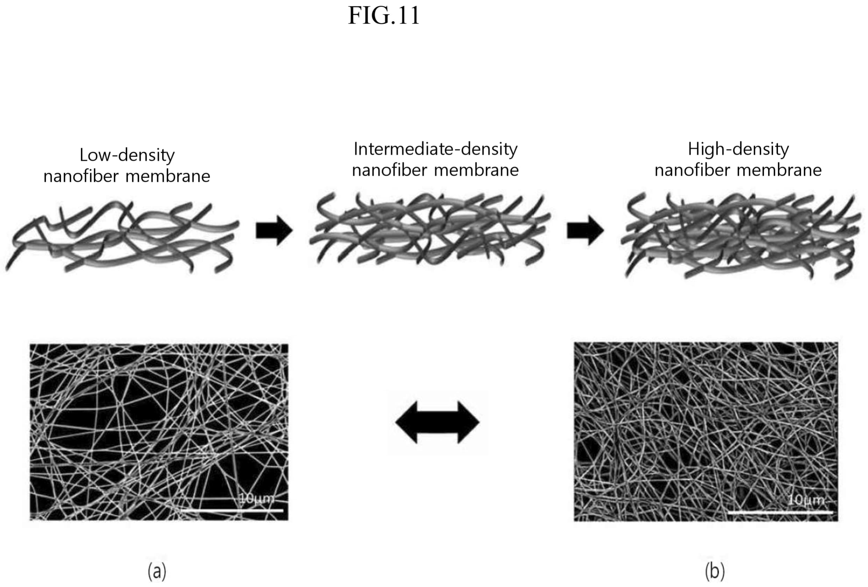

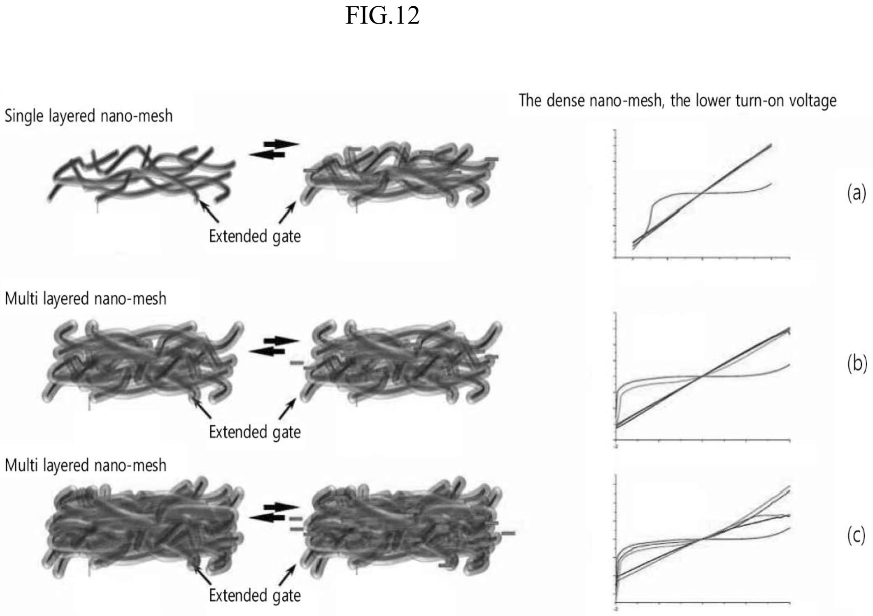

FIG. 11 exemplarily illustrates a nanofiber membrane according to a density variation, and FIG. 12 is a schematic diagram exemplarily illustrating voltage characteristics according to density changes of a nanofiber membrane having nanoelectrodes formed thereon. The nanofiber membrane according to an exemplary embodiment of the present disclosure may control a density of nanofibers by adjusting electrospinning characteristics. Such density control is intended to increase the surface area per unit volume, and for example, when the nanofiber membrane is manufactured with the same volume using thinner fibers, the surface area per unit volume becomes much larger, so if drugs are included inside the nanofiber membrane, a drug release rate becomes much faster, and if there are electrodes on the surface of the nanofiber membrane, a sensing rate or sensitivity of the electrodes may be much higher. Therefore, a high-density nanofiber membrane illustrated in (b) of FIG. 11 may have superior drug release characteristics and electrode characteristics compared to a low-density nanofiber membrane of (a) of FIG. 11.

Referring to FIG. 12, it can be seen that as the density of the nanofiber membrane increases, the sensing rate of the electrode increases. The single-layer nanofiber membrane as illustrated in (a) of FIG. 12 has a relatively high turn-on voltage, the multi-layered nanofiber membrane as illustrated in (b) of FIG. 12 exhibits a lower turn-on voltage, and the high-density multi-layered nanofiber membrane as illustrated in (c) of FIG. 12 exhibits the lowest turn-on voltage. As described above, the bio-implantable microfiber graft according to an exemplary embodiment of the present disclosure may effectively enhance the drug release characteristics and nanoelectrode characteristics of the microfiber graft by forming very thin nanofibers in a high-density multi-layer structure, bundle structure, or twisted structure in one aspect.

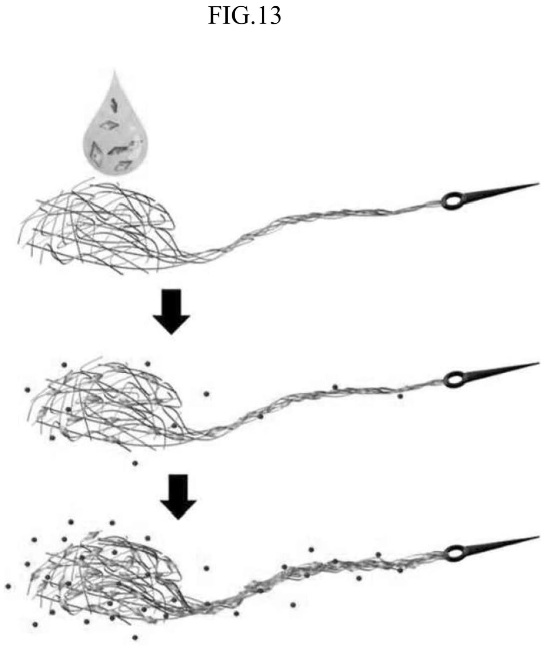

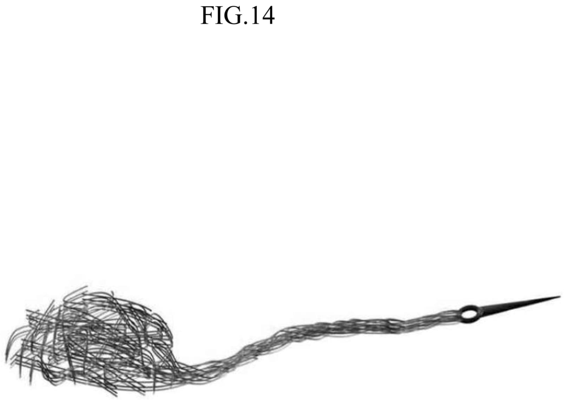

FIG. 13 exemplarily illustrates stem cell culture and material diffusion on the nanofiber membrane according to an exemplary embodiment of the present disclosure and FIG. 14 is a schematic diagram exemplarily illustrating a multi-layered structure of the bio-implantable microfiber graft. The method of manufacturing the bio-implantable microfiber graft according to an exemplary embodiment of the present disclosure may further include loading functional stem cells onto the porous nanofiber membrane. In an aspect, a stem cell culture fluid may be loaded onto the bio-implantable microfiber graft according to the present disclosure through various methods such as electrospinning, self-assembly, surface modification, and composite methods. When microfibers loaded with stem cells are implanted in the body, the stem cells consume healing factors released at the lesion site and further stimulate cells at the lesion site to release environment-responsive regenerative factors (paracrine effect), thereby further promoting recovery of the lesion site.

Referring to FIG. 14, in an aspect, the bio-implantable microfiber graft may be formed as a multi-layered structure having different characteristics, and the multi-layered structure may include a porous nanofiber membrane loaded with functional stem cells. The multiple layers of such microfibers may have different densities and strengths, and a cross-section of the microfibers may be formed so that the density and strength gradually change from one side to the other side. For example, when microfibers are implanted in a part such as a ligament, since the ligament is a connective tissue fiber that connects bone and muscle and is structurally arranged anisotropically, a part connected to the bone generally has high strength and a part connected to the muscle has flexible characteristics. Therefore, the bio-implantable microfiber graft according to the present disclosure may mimic such a structure by forming the nanofiber membranes having different characteristics in the multi-layered structure so that the density and strength of the microfibers gradually change.

According to an aspect of the present disclosure, a decellularized extracellular matrix-based hydrogel of a target organ may also be loaded on at least a portion of the surface of the microfiber. In order to load the decellularized extracellular matrix-based hydrogel, cells containing DNA are first removed through a decellularization process while maintaining the extracellular matrix of the organ to remove all components that may cause immune reactions, and then the decellularized extracellular matrix is processed in a powder form. Thereafter, the powdered decellularized extracellular matrix may be prepared into the hydrogel by adding crosslinking agents, bioactive substances, etc., to a hydrogel precursor solution and loaded onto the surface of the microfiber. In an aspect, since the decellularized extracellular matrix-based hydrogel includes tissue-derived cells that do not cause immune response, the decellularized extracellular matrix-based hydrogel may be loaded only on a site that contacts the tissue to be implanted.

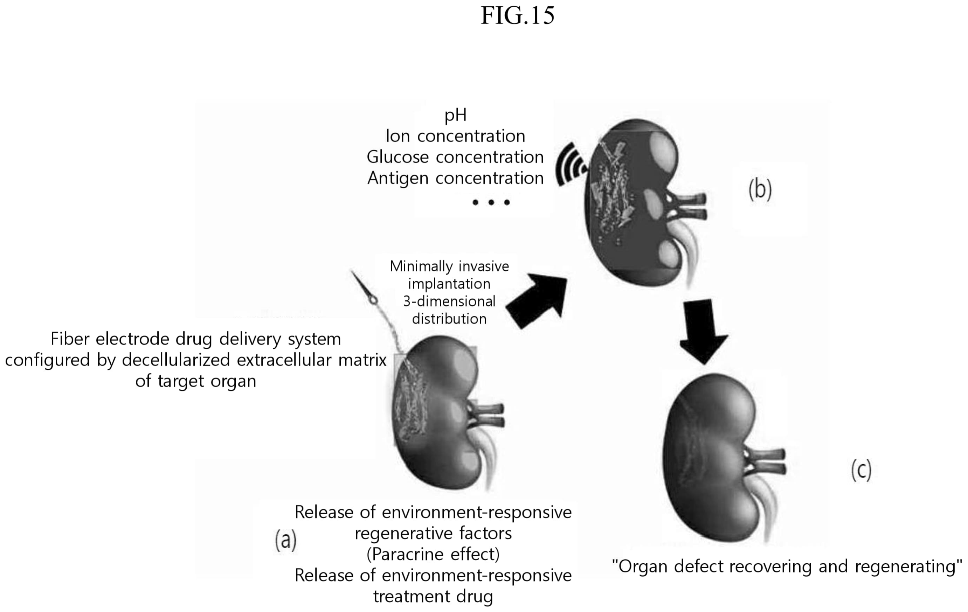

FIG. 15 exemplarily illustrates an operation of the bio-implantable microfiber graft according to an exemplary embodiment of the present disclosure. As described above, when one-dimensional microfibers are manufactured by forming the porous nanofiber membrane formed by electrospinning the nanofibers into the twisted structure, a bio-implantable microfiber graft having excellent mechanical strength while being flexible and excellent drug release characteristics due to a large surface area per unit volume may be provided. When such microfibers are implanted into a lesion site of tissue as surgical sutures as illustrated in (a) of FIG. 15, the microfiber graft may be implanted into the body in a minimally invasive manner while simultaneously providing a flexible yet high-strength scaffold. In addition, due to the porous and multi-layered structure of the porous nanofiber membrane according to the present disclosure, the drug release effect is naturally excellent. Meanwhile, by loading functional stem cells and decellularized extracellular matrix-based hydrogels onto the microfibers, environment-responsive regenerative factors may be released without rejection reactions to the microfiber graft, and accordingly, tissue cells at the lesion site may be effectively cultured. Furthermore, by loading the nanoelectrodes and the stimuli-responsive hydrogels onto the nanofiber membrane, as illustrated in (b) of FIG. 15, drugs may be released in response to pH, ion concentration, glucose concentration, and antigen concentration, and such drug release may be both stimulated and monitored by the nanoelectrodes. The bio-implantable microfiber graft according to an exemplary embodiment of the present disclosure enables the above-described actions to occur in combination, thereby recovering organ defects and regenerating cells and tissues as illustrated in (c) of FIG. 15.

Hereinabove, the present disclosure has been described with reference to the drawings and exemplary embodiments, but it is not meant that the protection scope of the present disclosure is limited by the drawings or exemplary embodiments, but those skilled in the art will understand that the present disclosure can be variously modified and changed without departing from the spirit and the scope of the present disclosure which are defined in the appended claims.

The present disclosure is described based on a series of functional blocks, but not limited to the aforementioned exemplary embodiments and the accompanying drawings, and it will be obvious to those skilled in the technical field to which the present disclosure pertains that various substitutions, modifications, and changes may be made within the scope without departing from the technical spirit of the present disclosure.

A combination of the exemplary embodiments is not limited to the aforementioned exemplary embodiment and various forms of combinations may be provided in addition to the aforementioned exemplary embodiments according to implementation and/or a need.

In the aforementioned exemplary embodiments, methods have been described based on flowcharts as a series of steps or blocks, but the methods are not limited to the order of the steps of the present disclosure and any step may occur in a step or an order different from or simultaneously as the aforementioned step or order. Further, it can be appreciated by those skilled in the art that steps shown in the flowcharts are not exclusive and other steps may be included or one or more steps do not influence the scope of the present disclosure and may be deleted.

The aforementioned exemplary embodiment includes various aspects of examples. All possible combinations for showing various aspects may not be described, but those skilled in the art may recognize that different combinations are possible. Accordingly, it will be intended that the present disclosure includes all other replacements, modifications, and changes which belong to the following claims.

## Claims

1. A method of manufacturing a bio-implantable microfiber graft, the method comprising:
manufacturing a porous nanofiber membrane based on a biocompatible polymer; and
manufacturing one-dimensional porous microfibers by forming the porous nanofiber membrane in a twisted structure.

2. The method of claim 1, wherein the porous nanofiber membrane or the microfibers are coated with perylene.

3. The method of claim 1, further comprising:
forming a nanoelectrode by depositing a conductive material on the porous nanofiber membrane.

4. The method of claim 3, wherein the nanoelectrode is formed in a two-dimensional multi-array structure on a surface of the porous nanofiber membrane, and
a plurality of nanoelectrodes included in the multi-array structure are arranged spaced apart from each other along a longitudinal direction of the microfibers when the porous nanofiber membrane is manufactured into microfibers.

5. The method of claim 1, further comprising:
forming the plurality of nanofiber membranes into a nanofiber bundle.

6. The method of claim 5, wherein the nanofiber bundle has a core having a first stiffness and a shell surrounding the core, which has a second stiffness different from the first stiffness.

7. The method of claim 6, wherein the core of the nanofiber bundle is formed of Polylactic Acid (PLA), and the shell is formed of Polycaprolactone (PCL).

8. The method of claim 5, wherein the nanofiber bundle is formed of hook-shaped nanofibers.

9. The method of claim 5, wherein the nanofiber bundle includes a main fiber and microfibers connected thereto.

10. The method of claim 5, wherein the nanofiber bundle is formed by mixing multi-layered nanofiber membranes and forming them in a rolled or twisted form.

11. The method of claim 1, wherein the nanofiber membrane is manufactured as a nanofiber membrane having an area equal to or greater than a predetermined critical area through multi-nozzles, so that the microfiber has a length equal to or greater than a predetermined critical length.

12. The method of claim 3, further comprising:
loading a stimuli-responsive hydrogel onto the nanoelectrode,
wherein the stimuli-responsive hydrogel contains a drug.

13. The method of claim 12, wherein the stimuli-responsive hydrogel is responsive to pH to release the drug.

14. The method of claim 12, wherein the stimuli-responsive hydrogel is responsive to an ion concentration to release the drug.

15. The method of claim 12, wherein the stimuli-responsive hydrogel is responsive to a glucose concentration to release the drug.

16. The method of claim 12, wherein the stimuli-responsive hydrogel is responsive to an antigen concentration to release the drug.

17. The method of claim 1, further comprising:
loading functional stem cells onto the porous nanofiber membrane.

18. The method of claim 17, wherein a cross-section of the microfiber formed by the porous nanofiber membrane loaded with the functional stem cells is formed such that density and strength gradually change from one side to the other side.

19. The method of any one of claims 1 to 18, wherein a decellularized extracellular matrix-based hydrogel of a target organ is loaded onto at least a portion of the surface of the microfiber.

20. A bio-implantable microfiber graft comprising: one-dimensional microfibers manufactured by forming a porous nanofiber membrane formed based on a biocompatible polymer into a nanofiber bundle and then forming the nanofiber bundle in a twisted structure.
